# EUROPEAN PATENT APPLICATION

(11) **EP 3 967 339 A1**
(43) Date of publication of application: **16.03.2022**
(21) Application number: 20801911.7
(22) Date of filing: 22.04.2020
(51) Int. Cl.: A61M 5/142, A61N 1/378, H02J 50/10, H02J 50/40, A61M 37/00

(54) **MEDICAL APPARATUS, EXTRACORPOREAL UNIT, TRANSMISSION SHEET, MEDICAL INSTRUMENT, AND POSITION DETECTION METHOD**

(30) Priority: 08.05.2019 JP 2019088521
(71) Applicant: Furukawa Electric Co., Ltd., Tokyo 100-8322 (JP)
(72) Inventor: NARA, Kazutaka, Tokyo 100-8322 (JP)
(74) Representative: SSM Sandmair
(86) International application number: PCT/JP2020/017398
(87) International publication number: WO 2020/226059

(57) **Abstract**

A medical device that makes it possible to easily check a given spot in a medical tool that is to be embedded in a body and is used, an external unit, a power transmission sheet, a medical tool, and a position detection method are provided. A medical device 1 includes a power transmitter 22 that transmits power without contact; a medical tool 10 that includes a power receiver that receives the power that is transmitted from the power transmitter 22 and that is to be embedded in a body and is used; and a light emitter 218 configured to, when a relative positional relationship between the power transmitter 22 and the medical tool 10 reaches a given state, notify that the relative positional relationship enters the given state.

## Description

### Field

The present disclosure relates to a medical device that detects a position of a medical tool that is embedded in the body of a subject and is used, an external unit, a power transmission sheet, a medical tool, and a position detection method.

### Background

A body-embedded medical device whose main unit is embedded in a body has been used as a medical tool in order to administer a chemical solution to the inside of the body. This medical tool reduces a strain on a patient whose has to have an injection frequently in order to administer a chemical solution. The medical tool has a soft part in the main unit into which an injection needle is inserted. The soft part is formed of, for example, silicone rubber, or the like. In the medical tool, a chemical solution is injected into a chemical solution container through the soft part. The chemical solution is conveyed to blood vessels via a catheter.

In such a medical tool, because a main unit is embedded in a body, it is difficult to specify the position of a soft part by sight from the outside of the body.

A medical tool to which the technique that is proposed in Patent Literature 1 is applied and whose soft part is made by mixing a luminescent agent that emits near-infrared luminescent light because of application of near-infrared excitation light into a resin material is conceivable. With such a medical tool, converting near-infrared fluorescence into visible light would possibly make it possible to specify the position of the soft part by sight.

### Citation List

### Patent Literature

Patent Literature 1: Japanese Patent No. 5958922 Summary

### Technical Problem

When a soft part is made by mixing a luminescent agent into a resin material, however, there is a possibility that the luminescent agent would coagulate, the amount of near-infrared luminesce that is emitted by the luminescent agent would decrease due to concentration quenching, and resultantly it would be difficult to specify the position of a soft part.

The medical tool having the soft part has been described here, and body-embedded medical devices having no soft part have a similar problem.

The disclosure was made in view of the above-described circumstances and an object of the disclosure is to provide a medical device that makes it possible to easily check a given site in a medical tool that is embedded in a body and is used, an external unit, a power transmission sheet, a medical tool, and a position detection method.

### Solution to Problem

To resolve the above problem and attain the object, a medical device according to the present disclosure includes: a power transmitter configured to transmit power without contact; a power receiver configured to receive the power that is transmitted from the power transmitter; a medical tool that includes any one of the power transmitter and the power receiver and that is to be embedded in a body and is used; and a notification unit configured to, when a relative positional relationship between the power transmitter and the power receiver reaches a given state in association with move of any one of the power transmitter and the power receiver, notify that the relative positional relationship enters the given state.

In the medical device according to the present disclosure, the notification unit is to, based on the power that is received by the power receiver, notify that the relative positional relationship enters the given state.

In the medical device according to the present disclosure, the notification unit is to, when a value of the power that is received by the power receiver exceeds a given threshold, notify that the relative positional relationship enters the given state.

In the medical device according to the present disclosure, the notification unit includes at least any one of a light emitter configured to notify that the relative positional relationship enters the given state by emitting light, an output unit configured to notify that the relative positional relationship enters the given state by outputting sound, and a display unit configured to make a notification by displaying information indicating that the relative positional relationship enters the given state.

In the medical device according to the present disclosure, the medical tool includes a main unit that is to be embedded in a body and is used; and a soft part of the main unit into which an injection needle for injecting a chemical solution is to be inserted, and the power receiver includes a first coil that is arranged around the soft part.

The medical device according to the present disclosure further includes an external unit including the power transmitter and the notification unit.

In the medical device according to the present disclosure, the external unit further includes a power source unit to supply power to the power transmitter; and a casing that houses the power source unit, and the power transmitter is detachable from the casing.

In the medical device according to the present disclosure, the power transmitter includes a sheet unit that is platy; a second coil that is arranged on one side of the sheet unit and that is for transmitting the power; and a connector that is arranged on another side of the sheet unit and that is configured to electrically connect the second coil and the power source unit.

In the medical device according to the present disclosure, the sheet unit has a hole through which an injection needle is injectable in the second coil.

In the medical device according to the present disclosure, the sheet unit further includes a hole that is formed in the vicinity of the second coil and into which an injection needle is injectable; and a cutout that is cut from an outer circumference of the hole to an outer edge of the sheet unit.

In the medical device according to the present disclosure, the sheet unit further includes an adhesive member that is attachable to a subject on any one of both surfaces of the sheet unit.

The medical device according to the present disclosure further includes a housing in which the sheet unit can be housed and that is attachable to a subject.

In the medical device according to the present disclosure, the housing houses the sheet unit such that the sheet unit is movably inside.

In the medical device according to the present disclosure, the housing includes a window that is formed on a side of a front surface and through which a position of the sheet unit is movable; and an adhesive member that is arranged on a side of a back surface and that is attachable to a subject.

In the medical device according to the present disclosure, the medical tool includes a measurement unit configured to measure a value of power; and a first communication unit configured to transmit the value of power that is measured by the measurement unit via the power receiver, the external unit further includes a second communication unit configured to receive the value of power that is transmitted from the first communication unit via the power transmitter, and the notification unit is configured to make a notification of information based on the value of power that is received by the second communication unit.

In the medical device according to the present disclosure, the external unit further includes a controller configured to control at least a notification unit, the medical tool further includes a recorder configured to record subject information that identifies a subject, the first communication unit is configured to transmit the subject information via the power receiver, and the controller is configured to, based on the subject information that is received via the second communication unit, control the power to be transmitted by the power transmitter.

In the medical device according to the present disclosure, the medical tool includes a main unit that is to be embedded in in a body and is used; and a soft part of the main unit into which an injection needle for injecting a chemical solution is to be inserted, the power receiver includes a first coil that is arranged around the soft part, and a plurality of the notification units are arranged at given intervals around the soft part and are configured to, based on the power that is received via the first coil, notify that the relative positional relationship enters the given state.

The medical device according to the present disclosure, further includes an external unit including the power transmitter and the notification unit. Further, the medical tool includes a main unit that is to be embedded in a body and is used; and a soft part of the main unit into which an injection needle for injecting a chemical solution is to be inserted, the power receiver includes a magnetic member that is arranged around the soft part; and a first coil that is wound around the magnetic member, the external unit further includes a casing that houses a power source unit configured to supply power to the power transmitter, the power transmitter includes a sheet unit that is platy; a second coil that is arranged on one side of the sheet unit and that is for transmitting power; and a connector that is arranged on another side of the sheet unit and that is configured to electrically connect the second coil and the power source unit, and the notification unit is arranged on the sheet unit and is configured to notify that the positional relationship enters the given state in a way that, in a case where the power is supplied to the second coil, the second coil is fixed to the magnetic member via a subject and a positional relationship between the second coil and the connector is changed relatively.

An external unit according to the present disclosure which detects a given spot in a medical tool that is to be embedded in a body and is used and that includes a power receiver configured to receive power that is transmitted without contact from outside, the external unit includes: a power transmitter configured to transmit the power without contact; and a notification unit configured to, when a relative positional relationship between the power transmitter and the power receiver reaches a given state in association with move of the external unit, notify that the relative positional relationship enters the given state.

A power transmission sheet according to the present disclosure that is detachable from an external unit configured to detect a given spot in a medical tool that is to be embedded in a body and is used and that includes a power receiver configured to receive power that is transmitted without contact from outside, includes: a sheet unit that is platy; a second coil that is arranged on one side of the sheet unit and that is for transmitting the power; and a connector that is arranged on another side of the sheet unit and that is configured to electrically connect the second coil and a power source unit of the external unit.

A medical tool according to the present disclosure that is to be embedded in a body and is used and that is configured to receive power that is transmitted without contact from an external unit, includes: a main unit that is to be embedded in a body and is used; a soft part of the main unit into which an injection needle for injecting a chemical solution is to be inserted; and a power receiver that includes a first coil that is arranged around the soft part and that is configured to receive the power via the first coil.

The medical tool according to the present disclosure further includes a plurality of notification units that are arranged at given intervals around the soft part and that is configured to, based on the power that is received via the first coil, notify that a relative positional relationship between the external unit and the power receiver enters a given state.

A position detection method according to the present disclosure that is executed by a medical device including a power transmitter configured to transmit power without contact; a power receiver configured to receive the power that is transmitted from the power transmitter; a medical tool that includes any one of the power transmitter and the power receiver and that is to be embedded in a body and is used; and an external unit that includes another one of the power transmitter and the power receiver, includes: in a case where the power transmitter transmits the power toward the power receiver with the external unit being moved, when a relative positional relationship between the power transmitter and the power receiver reaches a given state, notifying that the relative positional relationship enters the given state.

### Advantageous Effects of Invention

According to the disclosure, an effect that it is possible to easily check a given site in a medical tool that is embedded in a body and is used is achieved.

### Brief Description of Drawings

FIG. 1 is a schematic diagram illustrating a schematic configuration of a medical device according to a first embodiment of the disclosure.
FIG. 2 is a block diagram illustrating a functional configuration of the medical device according to the first embodiment of the disclosure.
FIG. 3 is a schematic diagram illustrating a schematic configuration of a medical tool according to the first embodiment of the disclosure.
FIG. 4 is a schematic diagram illustrating a schematic configuration of an external unit according to the first embodiment of the disclosure.
FIG. 5A is a schematic diagram illustrating a schematic configuration of a power transmitter according to the first embodiment of the disclosure.
FIG. 5B is a schematic diagram illustrating another schematic configuration of the power transmitter according to the first embodiment of the disclosure.
FIG. 5C is a schematic diagram illustrating another schematic configuration of the power transmitter according to the first embodiment of the disclosure.
FIG. 5D is a schematic diagram illustrating another schematic configuration of the power transmitter according to the first embodiment of the disclosure.
FIG. 6A is a diagram illustrating an overview of a position detection method that is executed by the medical device according to the first embodiment of the disclosure.
FIG. 6B is a diagram illustrating the overview of the position detection method that is executed by the medical device according to the first embodiment of the disclosure.
FIG. 6C is a diagram illustrating the overview of the position detection method that is executed by the medical device according to the first embodiment of the disclosure.
FIG. 6D is a diagram illustrating the overview of the position detection method that is executed by the medical device according to the first embodiment of the disclosure.
FIG. 7A is a schematic diagram illustrating a schematic configuration of a power transmitter according to Modification 1 of the first embodiment of the disclosure.
FIG. 7B is a schematic diagram illustrating a schematic configuration of a power transmitter according to Modification 2 of the first embodiment of the disclosure.
FIG. 7C is an enlarged view obtained by schematically enlarging part of an area A1 illustrate in FIG. 7B.
FIG. 7D is a schematic diagram for describing an overview of the power transmitter being attached according to Modification 2 of the first embodiment of the disclosure.
FIG. 7E is an enlarged view obtained by enlarging part of an area A2 illustrated in FIG. 7D.
FIG. 8 is a diagram schematically illustrating a state where a power transmitter according to Modification 3 of the first embodiment of the disclosure is worn on a subject.
FIG. 9 is a diagram schematically illustrating a perspective view of the power transmitter according to Modification 3 of the first embodiment of the disclosure.
FIG. 10 is a diagram schematically illustrating attachment of the power transmitter according to Modification 3 of the first embodiment of the disclosure.
FIG. 11 is a block diagram illustrating a functional configuration of a medical device according to a second embodiment of the disclosure.
FIG. 12 is a schematic diagram illustrating a schematic configuration of a medial tool according to the second embodiment of the disclosure.
FIG. 13 is a flowchart illustrating an overview of a position detection method that is executed by an external unit according to the second embodiment of the disclosure.
FIG. 14 is a schematic diagram illustrating a schematic configuration of a medical tool according to a third embodiment of the disclosure.
FIG. 15 is a block diagram illustrating a functional configuration of a medical device according to the third embodiment of the disclosure.
FIG. 16A is a schematic diagram illustrating a schematic configuration of a medial tool according to the third embodiment of the disclosure.
FIG. 16B is a schematic diagram illustrating a schematic configuration of another medial tool according to the third embodiment of the disclosure.
FIG. 17 is a schematic diagram illustrating a schematic configuration of a medical device according to a fourth embodiment of the disclosure.
FIG. 18 is a diagram schematically illustrating operations of the medical device according to the fourth embodiment of the disclosure.
FIG. 19 is a schematic diagram illustrating a schematic configuration of a medical device according to a fifth embodiment.
FIG. 20 is a block diagram illustrating a functional configuration of the medical device according to the fifth embodiment.

### Description of Embodiments

Modes for carrying out the disclosure will be described in detail below with the drawings. Note that the following embodiments do not limit the disclosure. Each of the drawings that are referred in the following description only schematically illustrate the shapes, sizes, and positional relationships such that the content of the disclosure is understandable. In other words, the disclosure is not limited to only the shapes, sizes, and positional relationships that are exemplified in each drawing. Furthermore, in the following description, a medical device that detects a given spot in a medical tool that is embedded in a body of a subject including a human and an animal and is used, an external unit, a reception device and a position detection method will be described in detail.

### First Embodiment

### Configuration of Medical Device

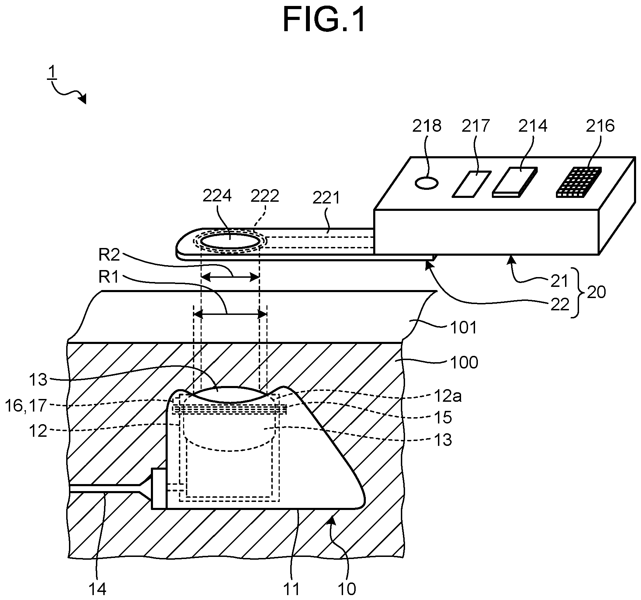

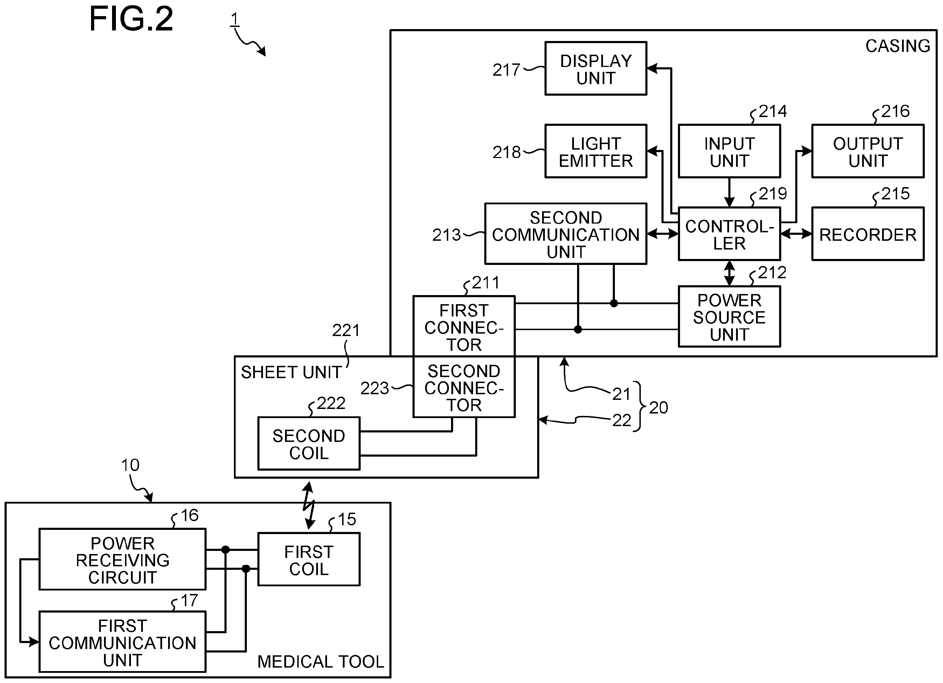

FIG. 1 is a schematic diagram illustrating a schematic configuration of a medical device according to a first embodiment. FIG. 2 is a block diagram illustrating a functional configuration of the medical device according to the first embodiment. A medical device 1 illustrated in FIG. 1 and FIG. 2 includes a medical tool 10 that is embedded in a living body 100 of a subject and is used and an external unit 20 that detects a given spot in the medical tool 10.

### Configuration of Medical Tool

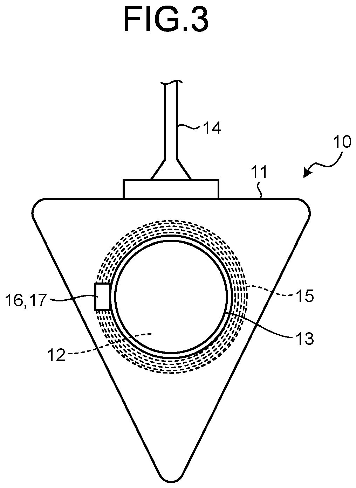

First of all, a detailed configuration of the medical tool 10 will be described. FIG. 3 is a schematic diagram illustrating a schematic configuration of the medical tool 10.

The medical tool 10 illustrated in FIGS. 1 to 3 is, for example, referred to as an under skin embedded port (CV port). As illustrated in FIGS. 1 to 3, a main unit 11 of the medical tool 10, is embedded in the living body 100 and is used.

The main unit 11 is a casing that is made of, for example, epoxy resin, or the like. As illustrated in FIGS. 1 to 3, the main unit 11 includes a chemical solution container 12, a soft part 13, a catheter 14, a first coil 15, a power receiving circuit 16, and a first communication unit 17.

The chemical solution container 12 is a chamber that stores a chemical temporarily. The chemical solution container 12 includes an opening 12a that is annular on the side of the outside of the body.

The soft part 13 is referred to as a so-called septum. The soft part 13 blocks the opening 12a of the chemical solution container 12. The soft part 13 is a soft cover (silicone diaphragm) that is made of, for example, silicone rubber and is arranged such that the soft part 13 is exposed from the main unit 11. The soft part 13 is cylindrical. The soft part 13 is a part into which an injection needle for injecting a chemical solution (transfusion) is insertable from a body surface 101 of the subject after the soft part 13 is embedded in the living body 100.

One end of the catheter 14 and the chemical solution container 12 communicate and the other end is inserted into a blood vessel not illustrated in the drawing, or the like. The catheter 14 conveys the chemical solution that is temporarily stored in the chemical solution container 12.

The first coil 15 is formed by multiple annular windings around an end face of the chemical solution container 12 on the side of the outside of the body and is arranged around the soft part 13. The first coil 15 is small and is mainly made of copper cables in order to increase power to be supplied and, in order to further reduce the weight, the first coil 15 may be made of aluminum cables. For example, the first coil 15 is formed in about 5 g. The first coil 15 is formed integrally with the main unit 11 by molding.

The power receiving circuit 16 receives power (induced electromotive force) that is generated in the first coil 15 and outputs a power reception result of the power reception to the first communication unit 17. In the first embodiment, the first coil 15 and the power receiving circuit 16 function as a power receiver.

The first communication unit 17 receives the power reception result that is input from the power receiving circuit 16 via the first coil 15 and transmits radio waves to the external unit 20. The power receiving circuit 16 and the first communication unit 17 may be formed integrally in an IC chip or may be formed separately. When a subject with the medical tool 10 being embedded in the body utilizes MRI, the IC chip would be exposed to a strong magnetic field. For this reason, the medical tool 10 may be configured to include a current detection function of detecting a current that flows through the first coil 15 and a shielding function, such as a switch, that electrically shields the first coil, the power receiving circuit 16, and the first communication unit 17. Such a configuration enables the IC chip to, when detecting a large current at or above a given threshold by the current detection function, have shielding from a current flowing into the first communication unit 17 by electrically shielding the first coil, the power receiving circuit 16, and the first communication unit 17 by the shielding function.

### Configuration of External Unit

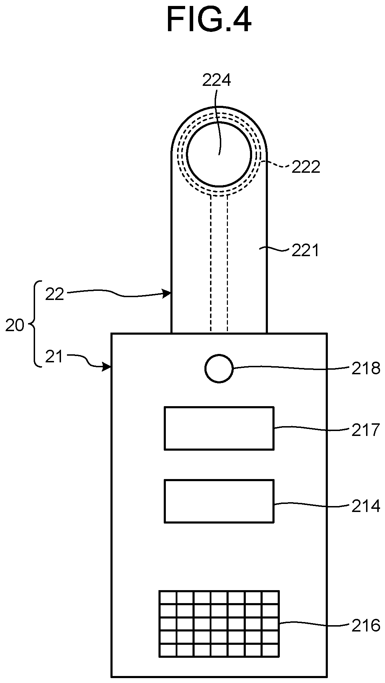

A schematic configuration of the external unit 20 will be described. FIG. 4 is a schematic diagram illustrating a schematic configuration of the external unit 20.

The external unit 20 illustrated in FIGS. 1, 2 and 4 detects a given spot in the medical tool 10 that is embedded in the living body 100 and is used. When a relative positional relationship between the first coil 15 of the medical tool 10 and a power transmitter to be described below reaches a given state, the external unit 20 notifies that the relative positional relationship between the first coil 15 and the power transmitter to be described below enters the given state. As illustrated in FIGS. 1, 2 and 4, the external unit 20 includes a casing 21 and a power transmitter 22 that is detachable from the casing 21. The casing 21 and the power transmitter 22 may be formed integrally.

### Configuration of Casing

A configuration of the casing 21 will be described. The casing 21 houses inside a circuit board on which a power source, an IC chip, etc., to be described below are mounted. The casing 21 supplies power to the power transmitter 22 and receives a power reception result of power received by the power transmitter 22. The casing 21 includes a first connector 211, a power source unit 212, a second communication unit 213, an input unit 214, a recorder 215, an output unit 216, a display unit 217, a light emitter 218, and a controller 219.

The first connector 211 is electrically connectable to the power transmitter 22 that is mounted on the casing 21 and supplies power that is input from the power source unit 212 to the power transmitter 22. The first connector 211 is configured using, for example, an electric female coupler.

Under the control of the controller 219, the power source unit 212 supplies power to the power transmitter 22 via the first connector 211. The power source unit 212 is configured using a battery, a booster circuit, etc.

Under the control of the controller 219, the second communication unit 213 outputs, to the controller 219, the power reception result (signal) that is received by the power transmitter via the first connector 211.

The input unit 214 receives an input of an operation of a user. The input unit 214 is realized using a button, a switch, a touch panel, or the like. The user herein is any one of a doctor, a nurse, a carer, and a patient who is the subject.

The recorder 215 records various programs to be executed by the external unit 20 and information. The recorder 215 is realized using a volatile memory and a nonvolatile memory.

The output unit 216 outputs sound under the control of the controller 219. The output unit 216 is realized using, for example, a speaker, or the like.

The display unit 217 displays given information under the control of the controller 219. The display unit 217 is realized using liquid crystals, organic electro luminescence (EL), or the like.

The light emitter 218 emits light under the control of the controller 219. The light emitter 218 is realized using a light emitting diode (LED).

The controller 219 controls each unit of which the external unit 20 consists. When the input unit 214 is pressed, the controller 219 controls the power source unit 212 and supplies power to the power transmitter 22 via the first connector 211. When a power reception result is input from the second communication unit 213, the controller 219 causes the light emitter 218 to emit light, thereby notifying that the relative positional relationship between the power transmitter 22 and the medical tool 10 enters the given state. Specifically, the controller 219 causes the light emitter 218 to emit light to notify that the relative positional relationship with the medical tool 10 enters the given state that is, for example, the state that a second coil 222 of the power transmitter 22 to be described below is positioned above the first coil 15 of the medical tool 10 based on the power of the power reception result from the second communication unit 213. The controller 219 is realized using a memory and a processor including hardware, such as a central processing unit (CPU).

### Configuration of Power Transmitter

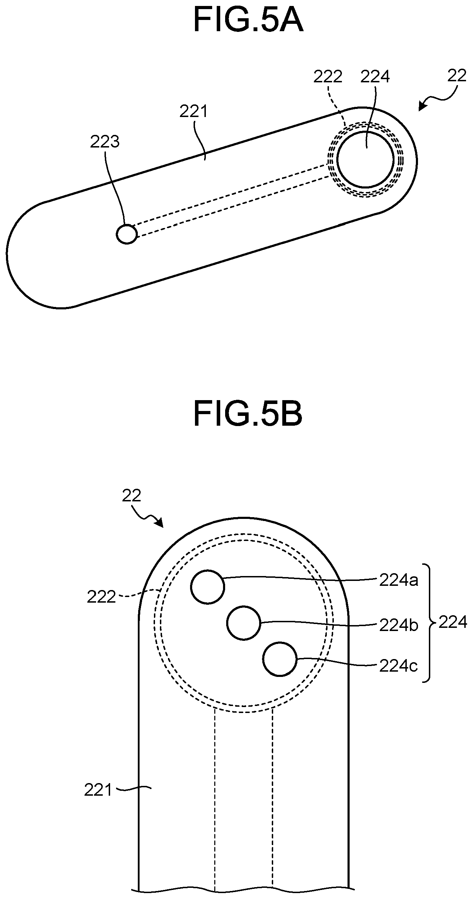

A configuration of the power transmitter 22 will be descried next. FIG. 5A is a schematic diagram illustrating a schematic configuration of the power transmitter 22. The power transmitter 22 has a function of transmitting power without contact.

The power transmitter 22 illustrated in FIGS. 1, 2 and 5 has a sheet unit 221, the second coil 222, a second connector 223, and a hole 224. In the first embodiment, the power transmitter 22 functions as a power transmission sheet. When an injection needle is inserted into the soft part 13, the injection needle may be inserted in the same spot many times. Thus, as illustrated in FIG. 1, in order not to have positions of insertion of the injection needle concentrate on one spot, it is preferable that an inner diameter dimension R2 of the hole 224 be designed slightly smaller than an outer diameter dimension R1 of the soft part 13. For example, a configuration in which the inner diameter dimension R2 of the hole 224 is smaller than the outer diameter dimension R1 of the soft part 13 by about ϕ1 mm is conceivable.

The sheet unit 221 is platy and both ends of the sheet unit 221 in its longitudinal direction are formed into arcs. The sheet unit 221 is realized using, for example, non-woven fabric, or the like. The sheet unit 221 is formed using two sheets of non-woven fabric with the second coil 222 and the second connector 223 interposed in between. Note that, in the first embodiment, a seal member on which an attachment member or an adhesive member that is attachable to the subject may be provided on a side of a back surface of the sheet unit 221 (on the side of the subject). In the first embodiment, a lubricant may be provided instead of the seal member on the side of the back surface of the sheet unit 221 (on the side of the subject) such that the sheet unit 221 can smoothly move on the body surface 101 of the subject.

The second coil 222 is annular and is arranged on one side of the sheet unit 221 in its longitudinal direction. The second coil 222 generates a magnetic flux according to the power that is input from the casing 21 via the second connector 223.

The second connector 223 is arranged on the other side of the sheet unit 221 in its longitudinal direction and electrically connects the second coil 222 and the power source unit 212 of the casing 21. The second connector 223 is configured using, for example, an electric male coupler.

The hole 224 is formed in the second coil 222. For example, the hole 224 is formed at the center of arrangement of the second coil 222. An injection needle is insertable through the hole 224. Note that the inner diameter of the second coil 222 and the diameter of the hole 224 are changeable as appropriate according to the diameter of the soft part 13.

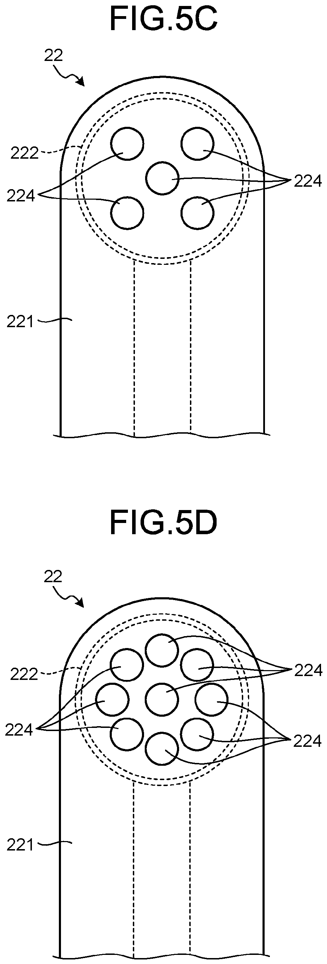

The hole 224 may consist of multiple holes as illustrated in FIGS. 5B, 5C and 5D. For example, when the hole 224 consists of three holes as in FIG. 5B, the user uses a hole 224a to insert an injection needle for the first time. The user then uses a hole 224b to insert the injection needle for the second time. Furthermore, the user uses a hole 224c to insert the injection needle for the third time. According to such a configuration, it is possible to effectively disperse positions in which an injection needle is inserted, which makes it possible to reduce deterioration of the soft part 13. Note that the number of holes 224 and the positions in which the holes 224 are formed are determined freely and are not limited.

Position Detection Method executed by Medical Device

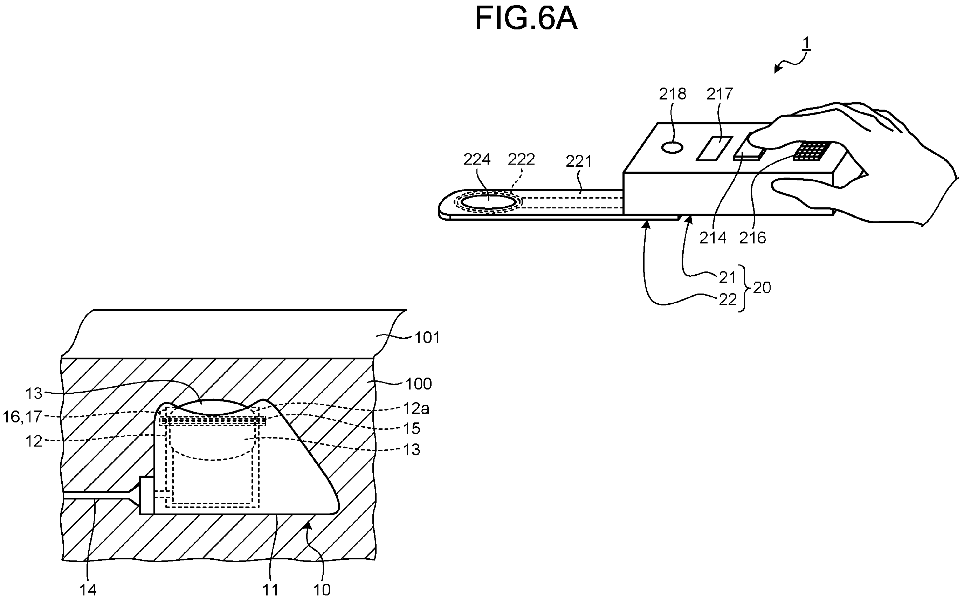

A position detection method that is executed by the medical device 1 will be described next. FIGS. 6A to 6D are diagrams schematically illustrating an overview of the position detection method that is executed by the medical device 1. FIGS. 6A to 6D illustrate the state where the medical tool 10 is embedded in the living body 100.

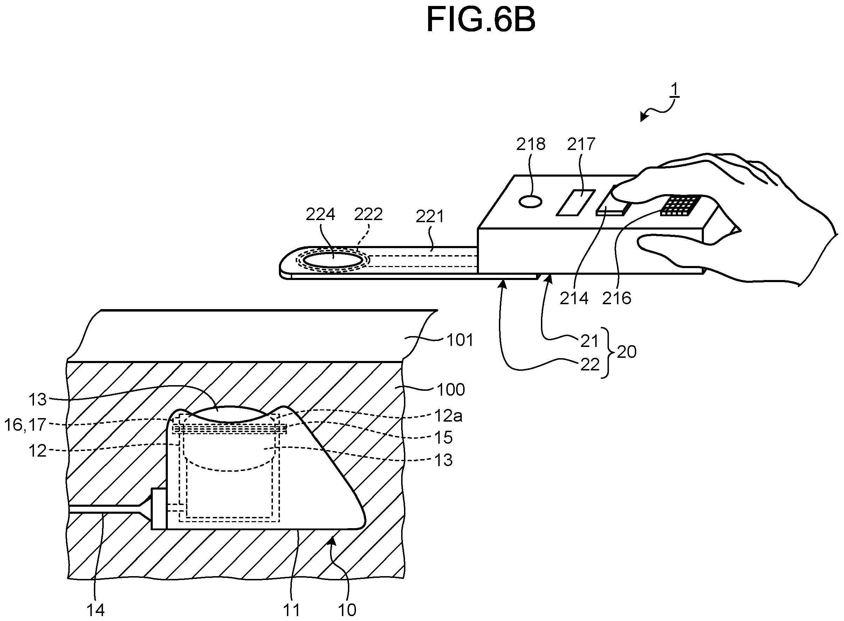

As illustrated in FIG. 6A, first of all, the user presses the input unit 214 of the external unit 20, thereby supplying power from the power source unit 212 of the casing 21 to the second coil 222 of the power transmitter 22. While searching for the medical tool 10 that is embedded in the living body 100, the user causes the power transmitter 22 of the external unit 20 to get close to the medical tool 10 (FIG. 6A → FIG. 6B). In this case, because power is supplied from the power source unit 212 via the second connector 223 and the first connector 211, the second coil 222 generates a magnetic flux.

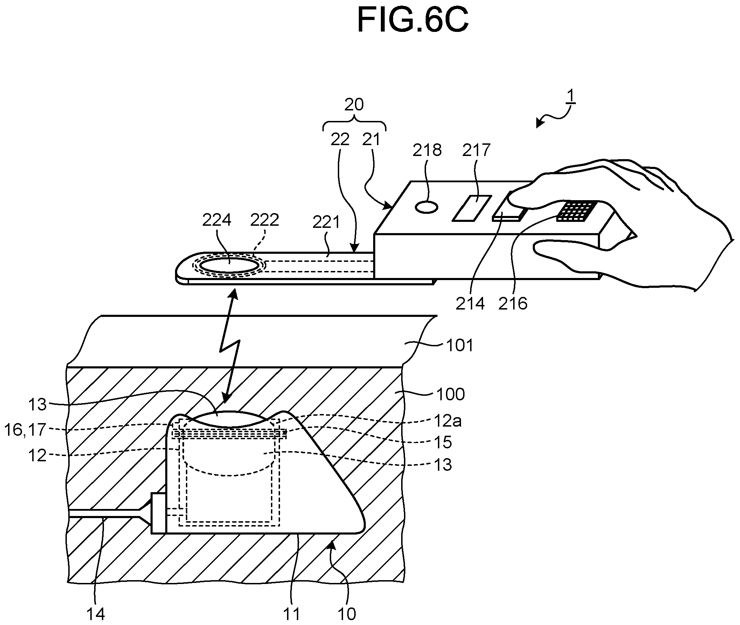

Subsequently, the user moves the external unit 20 further close to the medical tool 10 (FIG. 6B → FIG. 6C). Under the circumstances, when the relative positional relationship between the first coil 15 of the medical tool 10 and the second coil 222 reaches a given position, the light emitter 218 makes light emission indicating that the positional relationship reaches the given spot in the medical tool 10, thereby making a notification to the user. In this case, when the positional relationship between the first coil 15 of the medical tool 10 and the second coil 222 reaches a position in which the first coil 15 and the second coil 222 coincide vertically, the medical device 1 makes a notification to the user by emitting light. Specifically, the medical device 1 utilizes the aspect that the smaller the shift between the center of the first coil 15 of the medical tool 10 and the center of the second coil 222 is, the larger the supplied power is. In other words, in the medical device 1, when the center of the first coil 15 of the medical tool 10 and the center of the second coil 222 coincide, the current flowing into the second coil 222 reaches a power for driving the power receiving circuit 16 and the first communication unit 17 serving as part of the IC chip. Strictly, because the direction of depth is not constant depending on the patient, it is preferable that the external unit 20 have a function of adjusting the amount of power to be supplied. The first communication unit 17 of the medical tool 10 thus transmits a power reception result of reception of the power from the power transmitter 22 via the first coil 15. On receiving the power reception result via the power transmitter 22 and the second communication unit 213, the controller 219 causes the light emitter 218 to emit light, thereby notifying the user that the relative positional relationship between the first coil 15 of the medical tool 10 and the second coil 222 reaches the given position. As a result, the user is able to easily check the position of the soft part 13 in the medical tool 10 that is embedded in the living body 100 and is used. Specifically, the user is able to know that the soft part 13 is positioned under the hole 224 based on the site where the light emitter 218 emits light.

When the relative positional relationship between the first coil 15 of the medical tool 10 and the second coil 222 reaches the given position, the controller 219 may not only cause the light emitter 218 to emit light but also cause the output unit 216 to output sound, thereby make a notification to the user. Furthermore, the controller 219 may make a notification to the user by causing the display unit 217 to display information indicating that the second coil 222 reaches the soft part 13 in the medical tool 10.

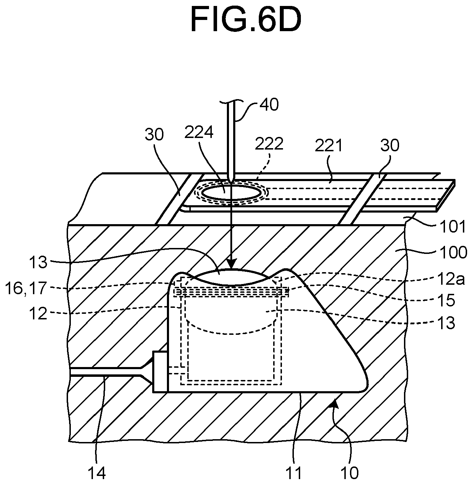

Thereafter, with the light emitter 218 emitting light, the user fixes the power transmitter 22 on the body surface 101 of the living body 100 in which the medical tool 10 is embedded using a tape 30, or the like, and detaches the power transmitter 22 from the casing 21 (FIG. 6C → FIG. 6D). The user then inserts an injection needle 40 using the hole 224 in the power transmitter 22 as a mark. Thus, the user is able to insert the injection needle 40 into the soft part 13 without dithering on the position of the soft part 13 of the medical tool 10 and thus is able to inject the chemical solution into the chemical solution container 12 easily. In FIG. 6D, the user fixes the power transmitter 22 right above the medical tool 10 using the tape 30, or the like, and, for example, an attachment member, such as a seal member or an adhesive member, may be provided on the back surface side of the power transmitter 22 to enable attachment to the body surface 101 of the living body 100. Furthermore, in FIG. 6D, the injection needle 40 may be inserted via the hole 224 of the power transmitter 22 without fixation of the power transmitter 22 to the living body 100.

According to the first embodiment described above, because, when the relative positional relationship between the first coil 15 of the medical tool 10 and the second coil 222 reaches the given position, the light emitter 218 emits light and thus notifies that the relative positional relationship between the first coil 15 of the medical tool 10 and the second coil 222 reaches the given position, it possible to easily check the position of the soft part 13 in the medical tool 10 that is embedded in the living body 100 and is used.

According to the first embodiment, because the power transmitter 22 of the external unit 20 is detachable from the casing 21 and thus is usable again even for another subject, it is possible to reduce the cost of introducing the medical device 1.

Furthermore, according to the first embodiment, because the first coil 15 is provided in a annular form around the soft part 13, notification made by the light emitter 218 by emitting light makes it possible to easily check the position (shape) of the soft part 13 in the medical tool 10.

Furthermore, according to the first embodiment, because the hole 224 is formed at the center of arrangement of the second coil 222 of the power transmitter 22, it is possible to easily insert the injection needle into the soft part 13 in the medical tool 10 with the relative positional relationship between the first coil 15 of the medical tool 10 and the second coil 222 having reached the given position.

The first embodiment may employ a configuration in which the first connector 211 and the second connector 223 are connected with a cable (not illustrated in the drawing). I the case of this configuration, the user is able to place the casing 21 on a table, or the like, and search for the medical tool 10 with the hand holding only the sheet unit 221.

### Modification 1 of the first embodiment

Modification 1 of the first embodiment will be described next. In the first embodiment described above, the hole 224 is formed at the center of the second coil 222 in the power transmitter 22; however, the power transmitter is not limited to this and the shape of the power transmitter may be changed as appropriate.

### Configuration of Power Transmitter

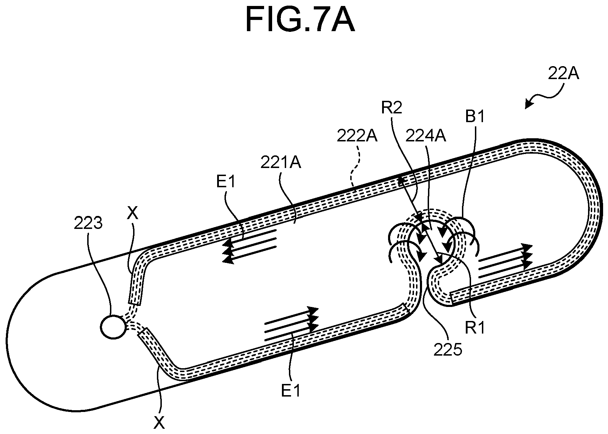

FIG. 7A is a schematic diagram illustrating a schematic configuration of the power transmitter according to Modification 1 of the first embodiment. A power transmitter 22A illustrated in FIG. 7A includes a sheet unit 221A, a second coil 222A, and a hole 224A instead of the sheet unit 221, the second coil 222 and the hole 224 of the power transmitter 22 according to the first embodiment described above. The power transmitter 22A further includes a cutout 225.

The sheet unit 221A is platy and both ends of the sheet unit 221A are formed into arcs. The sheet unit 221A is realized using, for example, non-woven fabric, or the like. The sheet unit 221A is formed using two sheets of non-woven fabric with the second coil 222A and the second connector 223 interposed in between.

The second coil 222A is arranged on the sheet unit 221A such that the flow of a current E1 separates from an opposed part so as not to cause canceling of an inversed magnetic flux B1 because of a current E1. Specifically, the second coil 222A is arranged along the edge of the sheet unit 221A. Particularly, in a part in which the hole 224A is formed, the sheet unit 221A may be formed such that a width R2 of the sheet unit 221A is equal to or larger than a diameter R1 of the hole 224A (R2≥R1). As illustrated in FIG. 7A, it is preferable that the second coil 222A excluding a part formed near the hole 224A be covered with a conductive sheet X. The conductive sheet X functions as a shield and thus is able to shield the part excluding the part formed near the hole 224A from external radiation of the magnetic field from the second coil 222A and is able to prevent the part from interacting with the first coil 15. For example, a metal sheet is conceivable as the conductive sheet X.

The hole 224A is formed in the vicinity of the second coil 222A. An injection needle is insertable through and removable from the hole 224A.

The cutout 225 is formed by making a cut from the hole 224A to the outer edge of the sheet unit 221A.

According to Modification 1 of the first embodiment described above, the user is able to remove the power transmitter 22A from the body surface of the subject with the injection needle being inserted into the medical tool 10 and this makes it possible to ensure hygiene.

According to Modification 1 of the first embodiment, when the user detects the position of the soft part 13 in the medical tool 10, the user inserts the injection needle into the medical tool 10 after temporarily fixing the sheet unit 221A on the subject with a tape, or the like. Thereafter, with the injection needle being left, the user detaches the external unit 20 including the sheet unit 221A from the subject via the cutout 225. The detached external unit 20 is usable many times and thus it is possible to further reduce the cost.

### Modification 2 of First Embodiment

Modification 2 of the first embodiment will be described next. In the above-described first embodiment, the shape of the second coil 222 in the power transmitter 22 is the same all the time regardless whether the power transmitter 22 is attached to the subject; however, the second coil 222 is not limited to this, and the shapes of the second coil and the power transmitter are changeable.

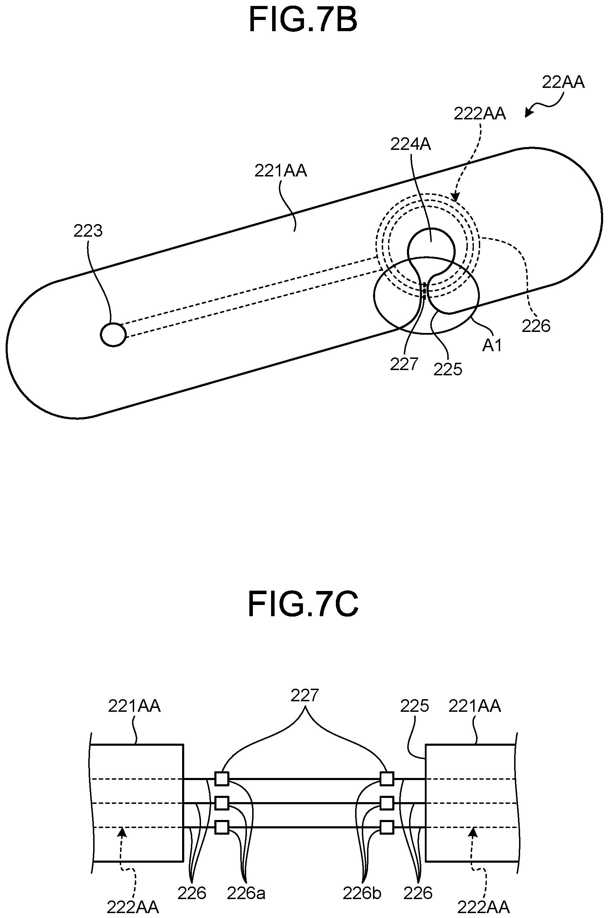

FIG. 7B is a schematic diagram illustrating a schematic configuration of a power transmitter according to Modification 2 of the first embodiment. FIG. 7C is an enlargement obtained by schematically enlarging part of an area A1 illustrated in FIG. 7B. A power transmitter 22AA illustrated in FIG. 7B and FIG. 7C includes a sheet unit 221AA and a second coil 222AA instead of the sheet unit 221, the second coil 222 and the hole 224 of the power transmitter 22 according to the first embodiment described above. The power transmitter 22AA further includes the hole 224A and the cutout 225 according to Modification 1 of the first embodiment described above.

The sheet unit 221AA is platy and both ends of the sheet unit 221AA in its longitudinal direction are formed into arcs. The sheet unit 221AA is realized using, for example, non-woven fabric, or the like. The sheet unit 221AA is formed using two sheets of non-woven fabric with the second coil 222AA and the second connector 223 interposed in between.

The second coil 222AA is annular and is arranged on one side of the sheet unit 221AA in its longitudinal direction. The second coil 222AA generates a magnetic flux according to power that is input from the casing 21 via the second connector 223. The second coil 222AA includes an annular part 226 and a joint 227 that electrically connects one end 226a of the annular part 226 and another end 226b of the annular part 226 and that is detachable. The joint 227 is made of an electric wire or an aluminum wire that electrically connects the end 226a and the other end 226b. The joint 227 need not be configured detachably from the end 226a and the other end 226b of the annular part 226 and, for example, a configuration in which a cut line is made at the center and the annular part 226 is disconnected may be employed or a configuration in which the end 226a and the other end 226b of the annular part 226 are insertable using a coupler, or the like, may be employed.

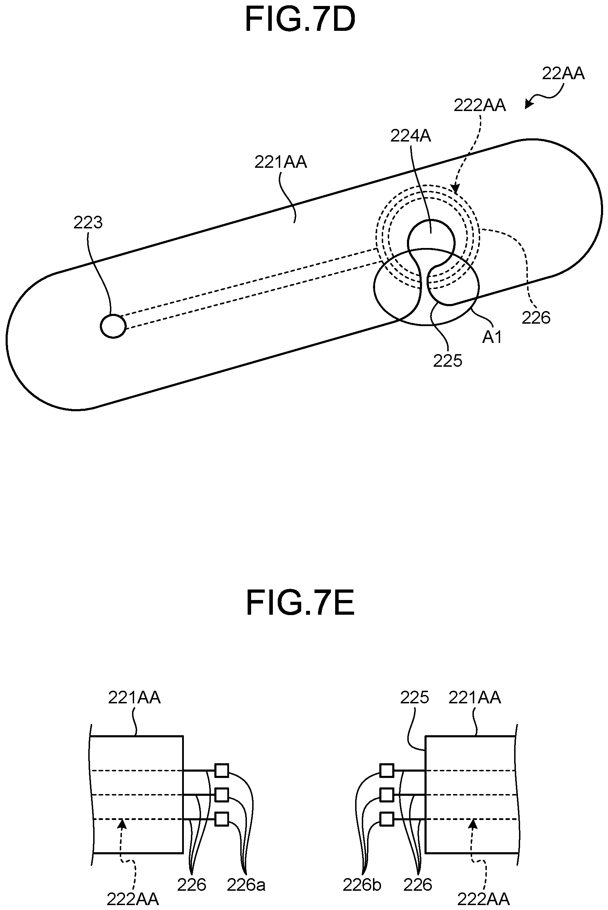

As for the power transmitter 22AA configured as described above, as illustrated in FIG. 7D and 7E, when a user detects the position of the soft part 13 in the medical tool 10, the user inserts an injection needle into the medical tool 10 after temporarily fixing the sheet unit 221AA to a subject with a tape, or the like. The user then detaches the joint 227 from the sheet unit 221AA. Accordingly, the user is able to detach the external unit 20 containing the sheet unit 221AA from the subject via the cutout 225 with the injection needle being left. The external unit 20 after being detached is usable many times, which makes it possible to reduce the cost.

According to Modification 2 of the first embodiment described above, because the joint 227 that electrically connects the end 226a and the other end 226b of the annular part 226 and that is detachable is provided in the second coil 222AA, it is possible to detached the external unit 20 including the sheet unit 221AA from the subject via the cutout 225 with an injection needle being left and thus it is possible to use the external unit 20 many times, which makes it possible to reduce the cost.

### Modification 3 of First Embodiment

Modification 3 of the first embodiment will be described next. In the first embodiment described above, the power transmitter 22 is attached to the external surface of the living body 100; however, the power transmitter is not limited to this, and the power transmitter may be attached to the external surface of the living body 100 by various methods.

### Configuration of Power Transmitter

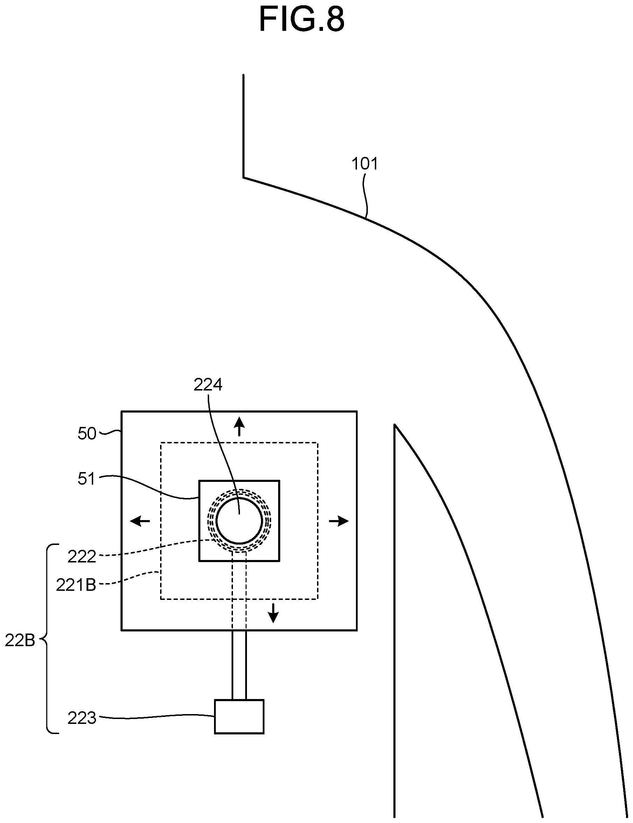

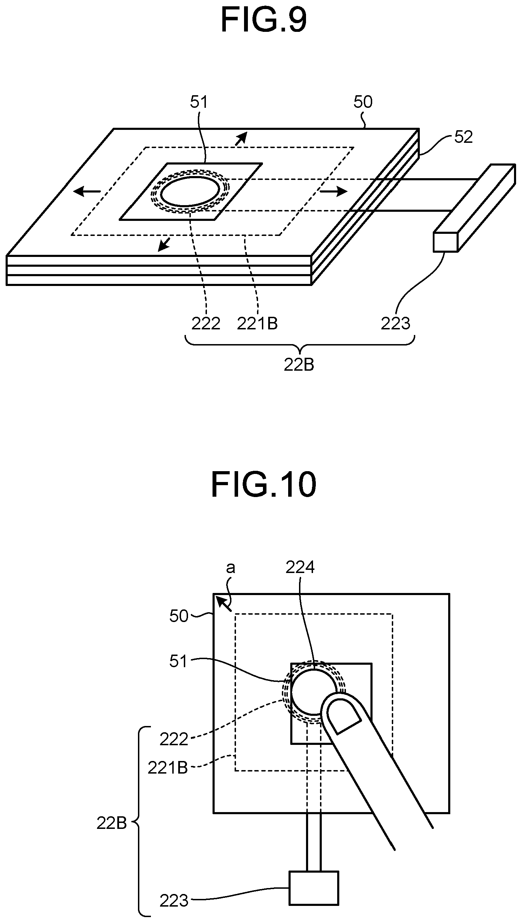

FIG. 8 is a diagram schematically illustrating a state where a power transmitter according to Modification 3 of the first embodiment is worn on a subject. FIG. 9 is a diagram schematically illustrating a perspective view of the power transmitter according to Modification 3 of the first embodiment.

A power transmitter 22B illustrated in FIG. 8 and FIG. 9 includes a sheet unit 221B that is platy and the second coil 222, the second connector 223 that is detachably and electrically connected to the above-described first connector 211 of the casing 21, and the hole 224, which are described above. Furthermore, in the state of being housed in a housing 50 that is attachable to a subject, the power transmitter 22B is worn on a subject 102.

The housing 50 houses the sheet unit 221B such that the sheet unit 221B is movable inside. The area of the upper surface of the housing 50 is larger than the area of the sheet unit 221B. The housing 50 includes, on the side of a front surface, a window 51 via which the position of the sheet unit 221B is operable and includes, on a side of a back surface, an adhesive member 52 that is attachable to an object. The housing 50 is formed using a small member with a small coefficient of friction, for example, poly carbonate, or the like.

In the state where the housing 50 is attached to the subject 102 and the power transmitter 22B configured as described above is housed in the housing 50, the power transmitter 22B is detached from the casing 21 right above the soft part 13 in the medical tool 10 by the position detection method of the first embodiment described above. Note that, because the user knows an approximate site where the medical tool 10 is embedded, the user attaches the housing 50 to the subject 102 in the vicinity of the site where the medical tool 10 is embedded. Thereafter, as illustrated in FIG. 10, after making a fine adjustment on the position of the sheet unit 221B via the window 51, the user fixes the power transmitter 22B in the housing 50 with a tape, or the like.

According to Modification 3 of the first embodiment described above, in the state of being housed in the housing 50, the power transmitter 22B is worn on the subject 102 and thus is attached with a small friction on the subject 102, which makes it possible to easily check the position of the soft part 13 in the medical tool 10.

### Second Embodiment

A second embodiment will be described next. In the first embodiment described above, the medical tool 10 transmits the received power to the external unit 20, thereby making a notification of the position of the given spot in the medical tool 10 and, in the second embodiment, a receiving circuit changes the content of notification based on the value of power that is received from a medical tool. The same reference numerals are assigned to the same components as those of the medical device 1 according to the first embodiment described above and detailed description thereof will be omitted.

### Configuration of Medical Device

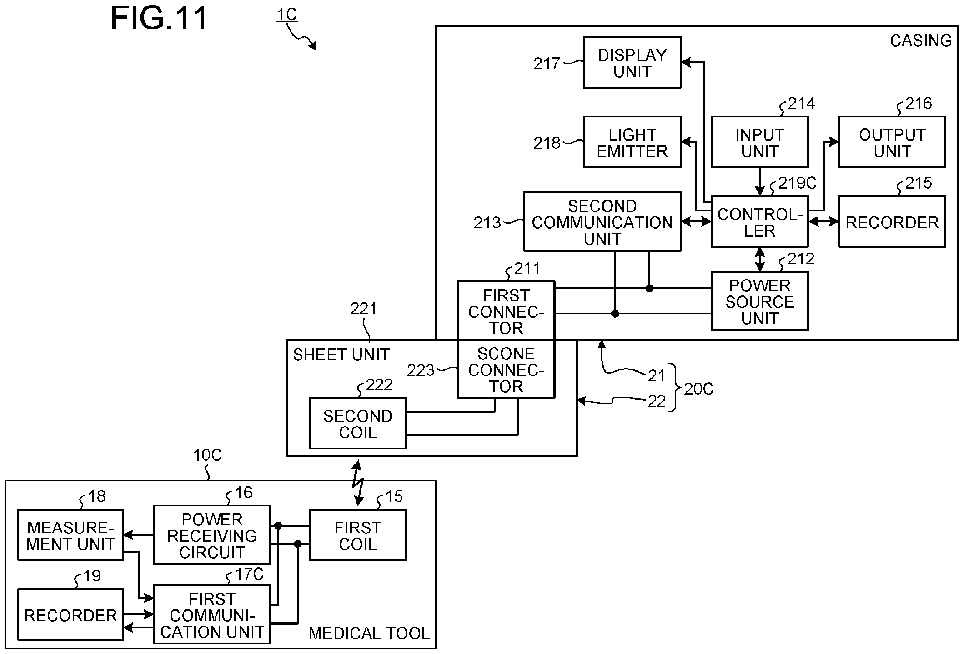

FIG. 11 is a block diagram illustrating a functional configuration of a medical device according to the second embodiment. A medical device 1C illustrated in FIG. 11 includes a medical tool 10C that is embedded in the living body 100 of the subject and is used and an external unit 20C that detects a given spot in the medical tool 10C.

### Configuration of Medical Tool

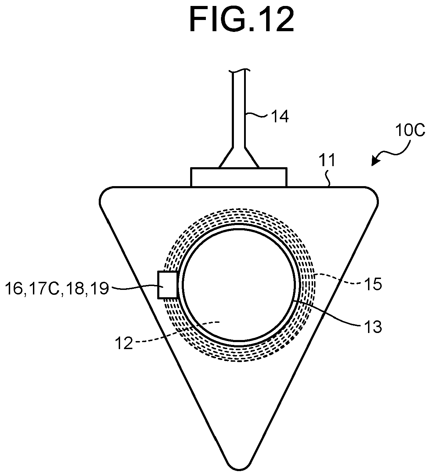

First of all, a configuration of the medical tool 10C will be described. FIG. 12 is a schematic diagram illustrating a schematic configuration of the medical tool 10C. The medical tool 10C illustrated in FIG. 11 and FIG. 12 includes a first communication unit 17C instead of the first communication unit 17 of the medical tool 10 according to the above-described first embodiment.

Furthermore, the medical tool 10C further includes a measurement unit 18 and a recorder 19.

The first communication unit 17C transmits a measurement result of measurement performed by the measurement unit 18 to the external unit 20C via the first coil 15. Furthermore, the first communication unit 17C transmits information that is recorded by the recorder to the external unit 20C via the first coil 15.

The measurement unit 18 measures a power value of power that is received by the power receiving circuit 16 and outputs the measurement result to the first communication unit 17C.

The recorder 19 records subject information on the subject in which the medical tool 10C is embedded. The subject information is, for example a name, a birth date, a weight, a doctor in charge, a type of applicable chemical solution, etc., for identifying the subject (patient). The recorder 19 is realized using an IC memory, or the like. Note that, in the second embodiment, the power receiving circuit 16, the first communication unit 17C, the measurement unit 18 and the recorder 19 may be formed integrally in an IC chip or may be formed separately.

### Configuration of External Unit

A configuration of the external unit 20C will be described next. The external unit 20C includes a controller 219C instead of the controller 219 according to the first embodiment described above.

The controller 219C controls each unit of which the external unit 20C consists. When the input unit 214 is pressed, the controller 219C controls a light emission state of light emission by the light emitter 218 based on a current value that is input via the second communication unit 213. The controller 219C is realized using a memory and a processor including hardware, such as a CPU.

### Position Detection Method executed by External Unit

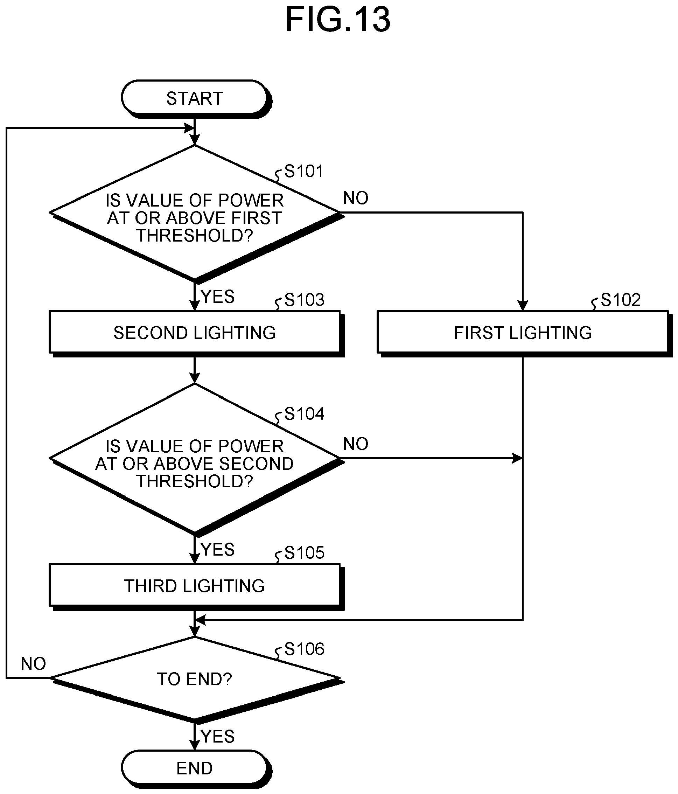

A position method that is executed by the medical device 1C will be described next. FIG. 13 is a flowchart illustrating an overview of the position detection method that is executed by the external unit. In FIG. 13, the state where the medical tool 10C is embedded in the living body 100 will be described.

As illustrated in FIG. 13, first of all, when a user presses the input unit 214 and, in a state where power is supplied from the power source unit 212 to the second coil 222 of the power transmitter 22, moves the sheet unit 221 close to the medical tool 10 that is embedded in the living body 100 (for example, see FIG. 6A → FIG. 6B), the controller 219 determines whether the value of power that is input via the second communication unit 213 and that is received by the medical tool 10C is at or above a first threshold (step S101). When the controller 219C determines that the value of power received by the medical tool 10C via the second communication unit 213 is not at or above the first threshold (NO at step S101), the external unit 20C moves to step S102 described below. When the controller 219 determines that the value of power received by the medical tool 10C via the second communication unit 213 is not at the first threshold or above (NO at step S101), the external unit 20C moves to step S102 to be described below. On the other hand, when the controller 219C determines that the value of power received by the medical tool 10C via the second communication unit 213 is at or above the first threshold (YES at step S101), the external unit 20C moves to step S103 to be described below.

At step S102, the controller 219C causes the light emitter 218 to emit light by first lighting. Specifically, the controller 219C causes the light emitter 218 to cause blue lighting. After step S102, the external unit 20C moves to step S106 to be described below.

At step S103, the controller 219C causes the light emitter 218 to emit light by second lighting. Specifically, the controller 219 causes the light emitter 218 to cause yellow or orange lighting.

Subsequently, in the state where power is supplied from the power source unit 212 to the second coil 222 of the power transmitter 22, when the sheet unit 221 is moved close to the medical tool 10 that is embedded in the living body 100 (see, for example, FIG. 6A → FIG. 6B → FIG. 6C described above), the controller 219C determines whether the value of power that is input via the second communication unit 213 and that is received by the medical tool 10C is at or above a second threshold (step S104). When the controller 219C determines the value of power that is input via the second communication unit 213 and that is received by the medical tool 10C is at or above the second threshold (YES at step S104), the external unit 20C moves to step S105. On the other hand, when the controller 219C determines that the value of power that is input via the second communication unit 213 and that is received by the medical tool 10C is not at or above the second threshold (NO at step S104), the external unit 20C moves to step S106 to be described below.

At step S105, the controller 219C causes the light emitter 218 to emit light by third lighting. Specifically, the controller 219C causes the light emitter 218 to cause green lighting. The site where the light emitter 218 causes green lighting indicates that the soft part 13 is positioned under the hole 224. Thus, when the user inserts the injection needle 40 using the hole 224 as a sign based on the site where the light emitter 218 causes green lighting, the user is able to insert the injection needle 40 into the soft part 13 without dithering on the position of the soft part 13 of the medical tool 10.

Subsequently, the controller 219C determines whether to end detecting a position of the soft part 13 in the medical tool 10C (step S106). Specifically, the controller 219C determines whether the user separates his/her fingers, or the like, from the input unit 214 and thus signals from the input unit 214 stop. When the controller 219C determines to end detecting a position of the soft part 13 in the medical tool 10C (YES at step S106), the external unit 20C ends the process. On the other hand, when the controller 219C determines not to end detecting a position of the soft part 13 in the medical tool 10C (step S106: NO), the external unit 20C returns to step S101 described above.

According to the second embodiment described above, because the light emitter 218 changes the state of light emission and makes a notification based on the value of power received by the second communication unit 213, it is possible to instinctively know the distance between the soft part 13 of the medical tool 10C and the second coil 222 of the power transmitter 22.

In the second embodiment, a configuration in which the controller 219C controls the power value of power that is supplied by the power source unit 212 based on the subject information that is input via the second communication unit 213 may be employed. According to this configuration, it is possible to supply power that is appropriate for each subject from the external unit 20C to the medical tool 10C.

### Third Embodiment

A third embodiment will be described next. In the first and second embodiments described above, the light emitter 218 of the external unit 20 or 20C performs light emission, or the like, and thus notifies that the relative positional relationship between the first coil 15 in the medical tool 10 or 10C and the second coil 222 enters the given state and, in the third embodiment, a notification is made by providing a medical tool with a notifying unit. Note that the same reference numerals are assigned to the same components as those of the medical device 1 according to the first embodiment described above and detailed description thereof will be omitted.

### Configuration of Medical Device

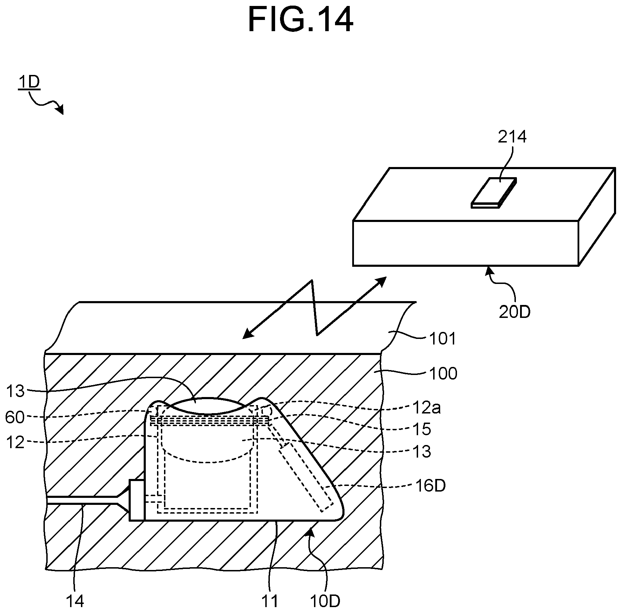

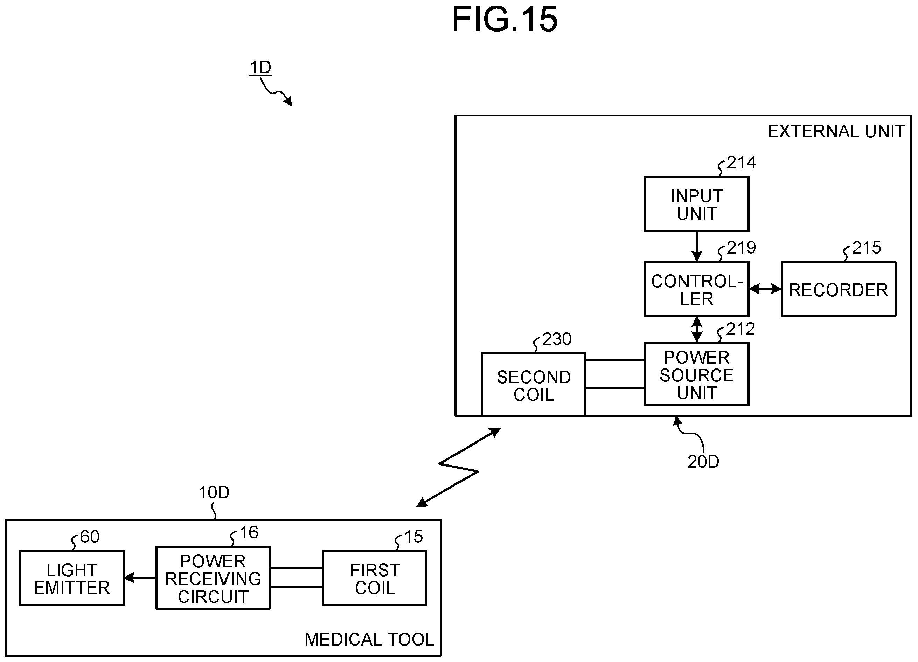

FIG. 14 is a schematic diagram illustrating a schematic configuration of a medical tool according to the third embodiment. FIG. 15 is a block diagram illustrating a functional configuration of the medical device according to the third embodiment.

### A medical device 1D illustrated in FIG. 14 and

FIG. 15 incudes a medical tool 10D that is embedded in the living body 100 of the subject and is used and an external unit 20D that detects a given spot in the medical tool 10.

### Configuration of Medical Tool

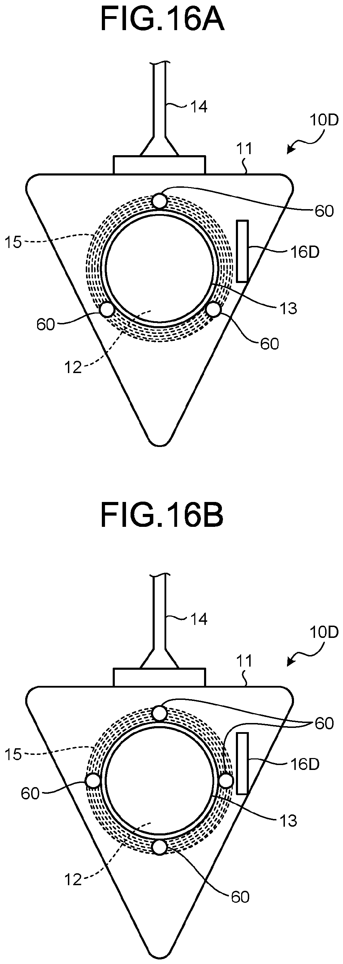

First of all, a detailed configuration of the medical tool 10D will be described. FIG. 16A is a schematic diagram illustrating a schematic configuration of the medical tool 10D.

The medical tool 10D illustrated in FIGS. 14 to 16A includes a plurality of light emitter 60 in addition to the components of the above-described first embodiment and the first communication unit 17 is omitted.

The light emitters 60 are arranged annularly at given intervals around the soft part 13. Specifically, the light emitters 60 are arranged in three spots around the soft part 13 annularly about the first coil 15 at every 120 degrees. The light emitters 60 emit light according to power that is received by the power receiving circuit 16. The light emitter 60 is configured using LEDs. Specifically, it is preferable that LEDs with high directionality be used for the light emitters 60. More specifically, it is preferable that red LEDs that emit light of a wavelength band of red be used because of transmission through a living body, such as a subject. Note that, as illustrated in FIG. 16B, the number of and arrangement of the light emitters 60 is changeable as appropriate.

### Configuration of External Unit

A configuration of the external unit 20D will be described next. As for the external unit 20D illustrated in FIG. 14 and FIG. 15, the second communication unit 213, the output unit 216, the display unit 217 and the light emitter 218 are omitted from the configuration of the casing 21 of the above-described first embodiment. Furthermore, the external unit 20D further includes a second coil 230.

The second coil 230 is annular, generates a magnetic flux according to power that is input from the power source unit 212 and transmits the power to the first coil. Power that is supplied by the second coil 230 enables the light emitter 60 to emit light even when the second coil 230 and the first coil 15 have a given distance in between, for example, a distance of around few tens of centimeters.

In the medical device 1D configured as described above, power is supplied from the external unit 20D and the light emitters 60 in the medical tool 10D emit light, thereby making a notification of the site in which the medical tool 10D is embedded. This enables the user to instinctively know the position and the center of the soft part 13 in the medical tool 10D from the outside of the subject.

According to the third embodiment described above, because the light emitters 60 in the medical tool 10D emits light and thus makes a notification, it is possible to instinctively know the position of the soft part 13 in the medical tool 10D from the outside of the subject.

In the third embodiment, the external unit 20D only transmits power to the medical tool 10D and, for example, as in the second embodiment, the medical tool 10D may be provided with the first communication unit 17C and the recorder 19 and the external unit 20D may be provided with the second communication unit 213 and the controller 219C, etc., thereby recording subject information and reading and writing subject information.

### Fourth Embodiment

A fourth embodiment will be described next. In the above-described first to third embodiments, when the electromagnetic induction system causes the relative positional relationship between the medical tool and the external unit to enter the given state, the light emitter emits light and thus notifies that the relative positional relationship between the medical tool and the external unit enters the given state and, in the fourth embodiment, a medical tool is magnetized and an external unit is fixed onto the surface of the subject, thereby notifying that the relative positional relationship between the medical tool and the external unit enters a given state. A configuration of the medical device according to the fourth embodiment will be described. The same reference numerals are assigned to the same components as those of the medical device 1 according to the above-described first embodiment and detailed description thereof will be omitted.

### Configuration of Medical Device

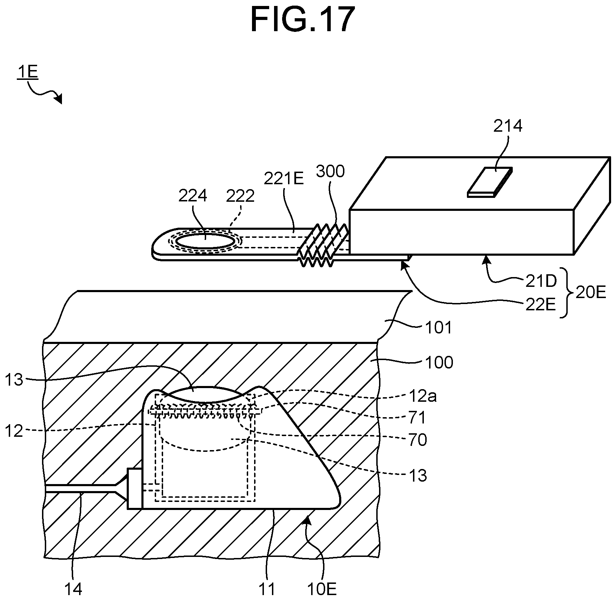

FIG. 17 is a schematic diagram illustrating a schematic configuration of the medical device according to the fourth embodiment. A medical device 1E illustrated in FIG. 17 includes a medical tool 10E that is embedded in the living body 100 of the subject and an external unit 20E that supplies power to the medical tool 10E.

### Configuration of Medical tool

First of all, a detailed configuration of the medical tool 10E will be described. The medical tool 10E illustrated in FIG. 17 includes a first coil 70 instead of the first coil 15 of the medical tool 10 according to the first embodiment described above and the power receiving circuit 16 and the first communication unit 17 are omitted. Furthermore, the medical tool 10E further includes a magnetic member 71 that is formed annularly on an end face of the chemical solution container 12 on the side of the outside of the body and that is arranged around the soft part 13. The first coil 70 is formed by windings around the magnetic member 71.

### Configuration of External Unit

A configuration of the external unit 20E will be described next. The external unit 20E illustrated in FIG. 17 includes a power transmitter 22E instead of the power transmitter 22 of the external unit 20 according to the above-described first embodiment. The power transmitter 22E includes a sheet unit 221E instead of the sheet unit 221 of the power transmitter 22 according to the above-described first embodiment. The sheet unit 221E further includes a change unit 300 that is formed into an accordion form in its longitudinal direction and that is extendable and is freely extendable.

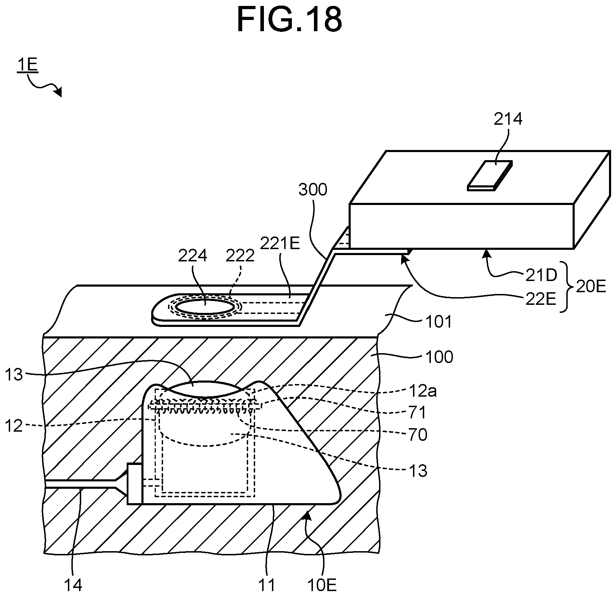

In the medical device 1E configured as described above, in the case where the user presses the input unit 214 of the external unit 20E and power is supplied from the power source unit 212 of the casing 21 to the second coil 222 of the power transmitter 22, when the user moves the external unit 20 close to the medical tool 10E that is embedded in the living body 100 while searching for the medical tool 10E and the relative positional relationship between the medical tool 10E and the external unit 20E enters a given state, the change unit 300 fixes onto the body surface 101 of the subject. Specifically, the second coil 222 is pulled by a magnetic force of the magnetic member 71 and accordingly is fixed onto the body surface 101 of the subject (refer to FIG. 18). Accordingly, the change unit 300 changes and thus the external unit 20E notifies the user in a pseudo manner of the relative relationship and accordingly the user is able to easily specify a given spot in the medical tool 10E, that is, the position of the soft part 13.

According to the fourth embodiment described above, because the second coil 222 is pulled by a magnetic force of the magnetic member 71 and accordingly is fixed onto the body surface 101 of the subject and the change unit 300 changes and thus makes a notification, it is possible to easily specify the position of the soft part 13 in the medical tool 10E.

### Fifth Embodiment

A fifth embodiment will be described next. In the first embodiment, the power source unit 212 is arranged in the casing 21 and, in the fifth embodiment, a power source unit is arranged in a power transmitter and the power transmitter and an external unit are capable of wireless bidirectional communication. The same reference numerals are assigned to the same components as those of the medical device 1 according to the first embodiment described above are thus detailed description thereof will be omitted.

### Configuration of Medical Device

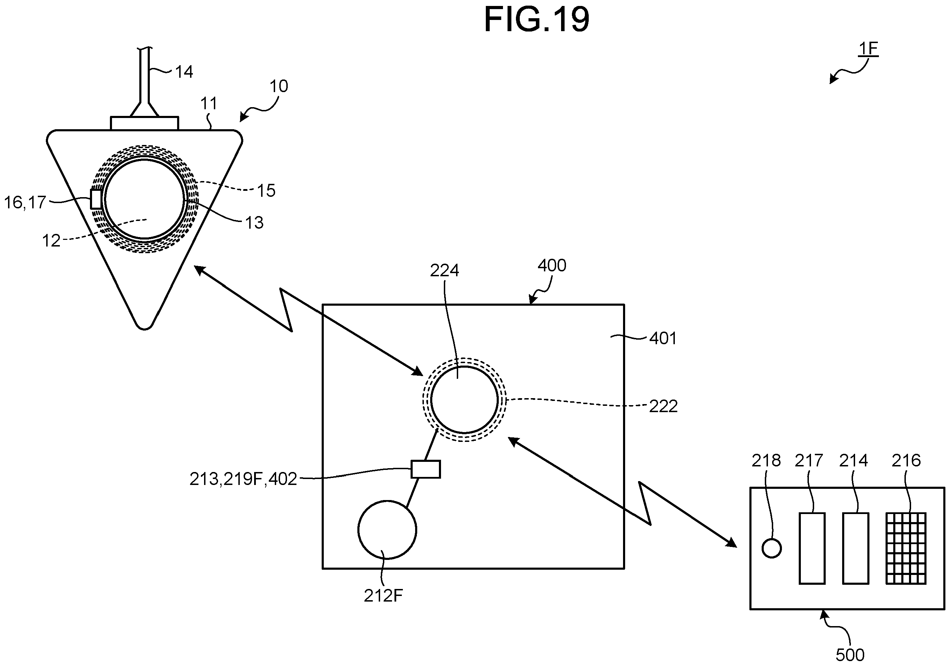

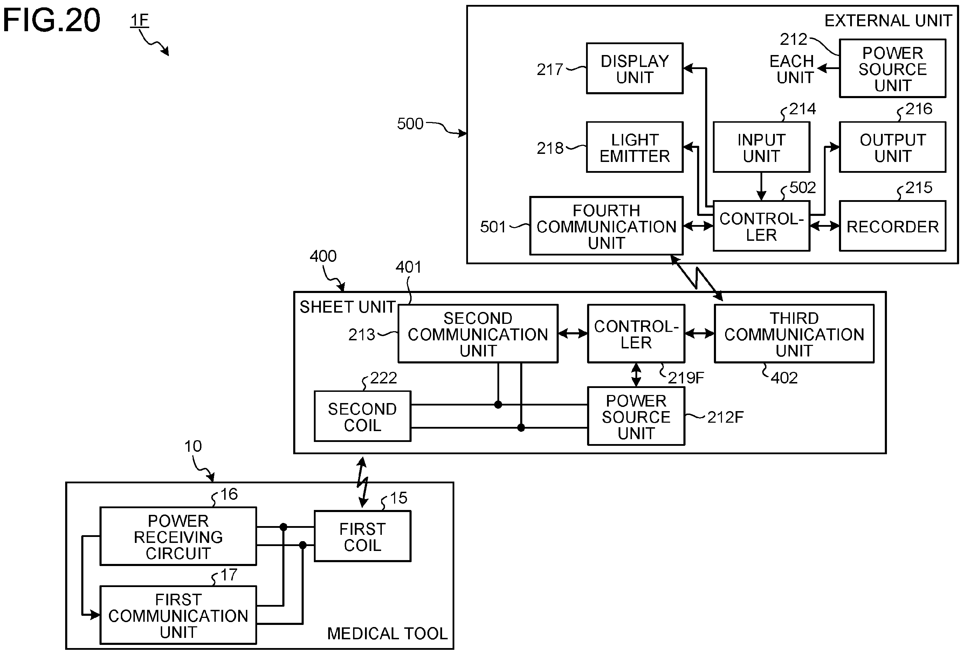

FIG. 19 is a schematic diagram illustrating a schematic configuration of a medical device according to the fifth embodiment. FIG. 20 is a block diagram illustrating a functional configuration of the medical device according to the fifth embodiment. A medical device 1F illustrated in FIG. 19 and FIG. 20 includes the medical tool 10 according to the above-described first embodiment, a power transmitter 400 that detects a given spot in the medical tool 10 and an external unit 500 that receives a detection result of detection by the power transmitter 400 and makes a notification.

### Configuration of Power Transmitter

First of all, a configuration of the power transmitter 400 will be described. The power transmitter 400 is rectangular and includes a sheet unit 401 that is attachable to a subject, the second coil 222, and the hole 224. Furthermore, the power transmitter 400 further includes a power source unit 212F, the second communication unit 213, a controller 219F, and a third communication unit 402.

The power source unit 212f is arranged on the sheet unit 401. The power source unit 212f is configured using, for example, an extremely thin button battery, or the like. Under the control of the controller 219F, the power source unit 212f supplies power to the second coil 222.

The controller 219F controls the power source unit 212F, the second communication unit 213 and the third communication unit 402. The controller 219F is realized using a memory and a processor including hardware, such as a CPU.

Under the control of the controller 219F, the third communication unit 402 performs bidirectional communication with a fourth communication unit 501 of the external unit 500 to be described below. Under the control of the controller 219F, the third communication unit 402 transmits a power reception result (signal) that is received by the second communication unit 213 to the fourth communication unit 501 of the external unit 500 to be described below. The third communication unit 402 is configured using, for example, a communication module capable of communication according to communication standards, such as Bluetooth (trademark) and Wi-Fi (Wireless Fidelity) (trademark).

### Configuration of External Unit

A configuration of the external unit 500 will be described. The external unit 500 includes the power source unit 212, the input unit 214, the recorder 215, the output unit 216, the display unit 217, the light emitter 218, the fourth communication unit 501, and a controller 502.

Under the control of the controller 502, the fourth communication unit 501 receives a power reception result that is transmitted from the third communication unit 402 of the power transmitter 400 and outputs the power reception result to the controller 502. The fourth communication unit 501 is configured using, for example, a communication module capable of communication according to communication standards, such as Bluetooth (trademark) and Wi-Fi.

The controller 502 controls each unit of which the external unit 500 consists. The controller 502 causes the light emitter 218 to emit light based on the power reception result that is input via the fourth communication unit 501 and that is transmitted from the third communication unit 402 of the power transmitter 400, thereby causing the light emitter 218 to notify that the relative positional relationship between the power transmitter 22 and the medical tool 10 enters a given state. The controller 502 is realized using a memory and a processor including hardware, such as a CPU. The controller 502 may not only cause the light emitter 218 to emit light but also cause the output unit 216 to output sound or cause the display unit 217 to display information.

According to the fifth embodiment described above, arranging the power source unit 212F in the power transmitter 400 makes it possible to omit wires, joint connectors, etc., for supplying power from the external unit 500 and thus make it possible to realize a simple configuration.

According to the first to fifth embodiments of the disclosure described above, when the electromagnetic induction system causes the relative positional relationship between the medical tool and the external unit to enter the given state, a notification is made to the user; however, the system is not limited to this, and a magnetic field resonance system, an electromagnetic wave reception system and an electric field coupling system are applicable.

In the first to fifth embodiments described above, the transmitter and the casing are independent from each other; however, the transmitter and the casing are not limited to this, and the transmitter and the casing may be integral.

In the first to fifth embodiments described above, the power transmitter and the casing are electrically connected and thus power is supplied to the power transmitter; however, power supply is not limited to this and, for example, power may be supplied from the casing to the power transmitter by wireless power supply.

In the first to fifth embodiments described above, the power source unit is arranged in the casing; however, the power source unit is not limited to this and, for example, the power source unit may be arranged in the power transmitter. In this case, a transmitting antenna is arranged in the power transmitter and a receiving antenna is arranged in the casing and, when the relative positional relationship between the medical tool and the external unit enters the given state, a signal notifying that the relative positional relationship between the medical tool and the external unit enters the given state may be transmitted from the transmitting antenna to the receiving antenna.

In the first to fifth embodiments described above, the second coil of the external unit transmits power to the first coil of the medical tool; however, the power transmission is not limited to this, and power may be transmitted from the first coil to the second coil.

It is possible to form various inventions by appropriately combining multiple components that are disclosed in the medical devices according to the first to fifth embodiments of the disclosure described above. For example, some components may be deleted from all the components described in the medical devices according to the first to fourth embodiments of the disclosure described above. Furthermore, the components that are described regarding the medical devices according to the first to fourth embodiments of the disclosure described above may be combined appropriately.

In the medical devices according to the first to fifth embodiments of the disclosure described above, the term "unit" mentioned above is replaceable with "means", "circuit", or the like. For example, the controller may be replaced with a control means or a control circuit.

Programs that the medical devices according to the first to fifth embodiments are caused to execute are provided in a manner that the programs are recorded in file data in an installable form or an executable form in a computer-readable recording medium, such as a CD-ROM, a flexible disk (FD), a CD-R, a DVD (Digital Versatile Disk), a USB medium, or a flash memory.

In the description of the flowcharts herein, the expressions such as "first of all", "then", and "subsequently", have been used to clearly indicate the temporal order of the processing between steps; however, the order of the processing required to carry out the disclosure is not uniquely determined by those expressions. That is, the order of the processing in the flowcharts described herein is changeable within a range without causing inconsistency.

Some embodiments of the application have been described in detail based on the drawings and the embodiments are exemplified only and it is possible to carry out the disclosure in, in addition to the modes described in the section of the disclosure of the invention, other modes in which various modifications and improvements are made based on knowledges of those skilled in the art.

### Reference Signs List

- 1, 1C, 1D, 1E, 1F: MEDICAL DEVICE

- 10, 10C, 10D, 10E: MEDICAL TOOL
- 11: BODY
- 12: CHEMICAL SOLUTION CONTAINER
- 12a: OPENING
- 13: SOFT PART
- 14: CATHETER
- 15: FIRST COIL
- 16B: POWER RECEIVING CIRCUIT
- 17, 17C: FIRST COMMUNICATION UNIT
- 18: MEASUREMENT UNIT
- 19: RECORDER
- 20, 20C, 20D, 20E, 500: EXTERNAL UNIT
- 21: CASING
- 22, 22A, 22AA, 22B, 22E, 400: POWER TRANSMITTER
- 30: TAPE
- 40: INJECTION NEEDLE
- 50: HOUSING
- 51: WINDOW
- 52: ADHESIVE MEMBER
- 60: LIGHT EMITTER
- 70: FIRST COIL
- 71: MAGNETIC MEMBER
- 100: LIVING BODY
- 101: BODY SURFACE
- 102: SUBJECT
- 211: FIRST CONNECTOR
- 212, 212F: POWER SOURCE UNIT
- 213: SECOND COMMUNICATION UNIT
- 214: INPUT UNIT
- 215: RECORDER
- 216: OUTPUT UNIT
- 217: DISPLAY UNIT
- 218: LIGHT EMITTER
- 219, 219C, 219f, 502: CONTROLLER
- 221, 221A, 221AA, 221B, 221E, 401: SHEET UNIT
- 222, 222AA, 230: SECOND COIL
- 223: SECOND CONNECTOR
- 224, 224A: HOLE
- 225: CUTOUT
- 226: ANNULAR PART
- 227: JOINT
- 300: CHANGE UNIT
- 402: THIRD COMMUNICATION UNIT
- 501: FOURTH COMMUNICATION UNIT

## Claims

1. A medical device comprising:
a power transmitter configured to transmit power without contact;
a power receiver configured to receive the power that is transmitted from the power transmitter;
a medical tool that includes any one of the power transmitter and the power receiver and that is to be embedded in a body and is used; and
a notification unit configured to, when a relative positional relationship between the power transmitter and the power receiver reaches a given state in association with move of any one of the power transmitter and the power receiver, notify that the relative positional relationship enters the given state.

2. The medical device according to claim 1, wherein the notification unit is configured to, based on the power that is received by the power receiver, notify that the relative positional relationship enters the given state.

3. The medical device according to claim 2, wherein the notification unit is configured to, when a value of the power that is received by the power receiver exceeds a given threshold, notify that the relative positional relationship enters the given state.

4. The medical device according to any one of claims 1 to 3, wherein the notification unit includes at least any one of a light emitter configured to notify that the relative positional relationship enters the given state by emitting light, an output unit configured to notify that the relative positional relationship enters the given state by outputting sound, and a display unit configured to make a notification by displaying information indicating that the relative positional relationship enters the given state.

5. The medical device according to any one of claims 1 to 4, wherein the medical tool includes
a main unit that is to be embedded in a body and is used; and
a soft part of the main unit into which an injection needle for injecting a chemical solution is to be inserted, and
the power receiver includes a first coil that is arranged around the soft part.

6. The medical device according to any one of claims 1 to 5, further comprising an external unit including the power transmitter and the notification unit.

7. The medical device according to claim 6, wherein the external unit further includes
a power source unit configured to supply power to the power transmitter; and
a casing that houses the power source unit, and
the power transmitter is detachable from the casing.

8. The medical device according to claim 7, wherein the power transmitter includes
a sheet unit that is platy;
a second coil that is arranged on one side of the sheet unit and that is for transmitting the power; and
a connector that is arranged on another side of the sheet unit and that is configured to electrically connect the second coil and the power source unit.

9. The medical device according to claim 8, wherein the sheet unit has a hole through which an injection needle is injectable in the second coil.

10. The medical device according to claim 9, wherein the sheet unit further includes
a hole that is formed in the vicinity of the second coil and into which an injection needle is injectable; and
a cutout that is cut from an outer circumference of the hole to an outer edge of the sheet unit.

11. The medical device according to any one of claims 8 to 10, wherein the sheet unit further includes an adhesive member that is attachable to a subject on any one of both surfaces of the sheet unit.

12. The medical device according to any one of claims 8 to 11, further comprising a housing in which the sheet unit can be housed and that is attachable to a subject.

13. The medical device according to claim 12, wherein the housing houses the sheet unit such that the sheet unit is movably inside.

14. The medical device according to claim 13, wherein the housing includes
a window that is formed on a side of a front surface and through which a position of the sheet unit is movable; and
an adhesive member that is arranged on a side of a back surface and that is attachable to a subject.

15. The medical device according to claim 6, wherein the medical tool includes
a measurement unit configured to measure a value of power; and
a first communication unit configured to transmit the value of power that is measured by the measurement unit via the power receiver,
the external unit further includes a second communication unit configured to receive the value of power that is transmitted from the first communication unit via the power transmitter, and
the notification unit is configured to make a notification of information based on the value of power that is received by the second communication unit.

16. The medical device according to claim 15, wherein the external unit further includes a controller configured to control at least a notification unit,
the medical tool further includes a recorder configured to record subject information that identifies a subject,
the first communication unit is configured to transmit the subject information via the power receiver, and
the controller is configured to, based on the subject information that is received via the second communication unit, control the power to be transmitted by the power transmitter.

17. The medical device according to claim 1, wherein the medical tool includes
a main unit that is to be embedded in in a body and is used; and
a soft part of the main unit into which an injection needle for injecting a chemical solution is to be inserted,
the power receiver includes a first coil that is arranged around the soft part, and
a plurality of the notification units are arranged at given intervals around the soft part and are configured to, based on the power that is received via the first coil, notify that the relative positional relationship enters the given state.

18. The medical device according to claim 1, further comprising an external unit including the power transmitter and the notification unit,
wherein the medical tool includes
a main unit that is to be embedded in a body and is used; and
a soft part of the main unit into which an injection needle for injecting a chemical solution is to be inserted,
the power receiver includes
a magnetic member that is arranged around the soft part; and
a first coil that is wound around the magnetic member,
the external unit further includes a casing that houses a power source unit configured to supply power to the power transmitter,
the power transmitter includes
a sheet unit that is platy;
a second coil that is arranged on one side of the sheet unit and that is for transmitting power; and
a connector that is arranged on another side of the sheet unit and that is configured to electrically connect the second coil and the power source unit, and
the notification unit is arranged on the sheet unit and is configured to notify that the positional relationship enters the given state in a way that, in a case where the power is supplied to the second coil, the second coil is fixed to the magnetic member via a subject and a positional relationship between the second coil and the connector is changed relatively.

19. An external unit configured to detect a given spot in a medical tool that is to be embedded in a body and is used and that includes a power receiver configured to receive power that is transmitted without contact from outside, the external unit comprising:
a power transmitter configured to transmit the power without contact; and
a notification unit configured to, when a relative positional relationship between the power transmitter and the power receiver reaches a given state in association with move of the external unit, notify that the relative positional relationship enters the given state.

20. A power transmission sheet that is detachable from an external unit configured to detect a given spot in a medical tool that is to be embedded in a body and is used and that includes a power receiver configured to receive power that is transmitted without contact from outside, the power transmission sheet comprising:
a sheet unit that is platy;
a second coil that is arranged on one side of the sheet unit and that is for transmitting the power; and
a connector that is arranged on another side of the sheet unit and that is configured to electrically connect the second coil and a power source unit of the external unit.

21. A medical tool that is to be embedded in a body and is used and that is configured to receive power that is transmitted without contact from an external unit, the medical tool comprising:
a main unit that is to be embedded in a body and is used;
a soft part of the main unit into which an injection needle for injecting a chemical solution is to be inserted; and
a power receiver that includes a first coil that is arranged around the soft part and that is configured to receive the power via the first coil.

22. The medical tool according to claim 21, further comprising a plurality of notification units that are arranged at given intervals around the soft part and that is configured to, based on the power that is received via the first coil, notify that a relative positional relationship between the external unit and the power receiver enters a given state.

23. A position detection method that is executed by a medical device including a power transmitter configured to transmit power without contact; a power receiver configured to receive the power that is transmitted from the power transmitter; a medical tool that includes any one of the power transmitter and the power receiver and that is to be embedded in a body and is used; and an external unit that includes another one of the power transmitter and the power receiver, the method comprising:
in a case where the power transmitter transmits the power toward the power receiver with the external unit being moved, when a relative positional relationship between the power transmitter and the power receiver reaches a given state, notifying that the relative positional relationship enters the given state.
